# EUROPEAN PATENT APPLICATION

(11) **EP 0 524 610 A1**
(43) Date of publication of application: **27.01.1993**
(21) Application number: 92112479.8
(22) Date of filing: 21.07.1992
(51) Int. Cl.: C12M 1/24

(54) **Culture flask**

(30) Priority: 22.07.1991 US 733501
(71) Applicant: Becton Dickinson and Company, Franklin Lakes, New Jersey 07417-1880 (US)
(72) Inventor: Tyndorf, Tadeusz A., Manalapan Township, NJ 07726 (US); Chu, I-Hsi D., West Orange, NJ 07052 (US)
(74) Representative: Selting, Günther, Dipl.-Ing.

(57) **Abstract**

A large flat surfaced blow molded culture flask is provided with a large flat cell culture surface. The flask is configured not only to provide the large surface for cell culture development, but also to provide ready accessibility to this surface through the large flask opening without interference. The blow molded structure of the flask eliminates seams, and therefore, leaks and potential contamination from biohazardous virus and/or bacteria. The top and bottom surface configuration of the flask allows for holding individual flasks in a stacked formation without any slippage to provide increased capacity in a controlled environment for cell culture development. The entire flask is transparent for easy examination of cell development.

## Description

### BACKGROUND STATEMENT OF THE INVENTION

This invention relates generally to culture flasks for use in the laboratory environment. More particularly, this invention relates to such flasks for placement in a room designed to provide a deliberate environment for the development of cells and to great quantities of such flasks arranged on shelves in such an environmentally arranged room, so that great quantities of such cells can be cultured simultaneously. Even more particularly, this invention relates to a specific culture flask having arranged on the top and bottom surfaces thereof mating arrangements which provide for stable stacking of a large number of such flasks on top of each other in the cell culture development environment.

As is well known, it has become increasingly important to provide for the culturing of a great many cells for examination purposes. This is particularly true with the advance of instrumentation for examining developed cultured cells and because of a variety of diseases which are properly and effectively examined through culture cell growth. Because of this, it becomes increasingly important to not only develop cultured cells, but to be able to develop a great many such cells in each individual container and to provide as many containers as possible in a specific environmental room for that purpose, so that a great many cells may be developed and produced for examination purposes.

Thus, petri dishes, tubes and culture flasks have been gradually increased in use over a period of years with culture flasks being utilized more and more because a larger quantities of cells may be produced in each container. Problems concerning the use of a flask for developing and culturing cells includes the ability to have access to the actual cells being developed in each container. It is important, from time to time, during the development and/or culturing period to be able to examine individual cell development for examining the status of the culturing procedure taking place in the container of interest.

Representative prior art patents include, for example, U.S. Patent 3,726,764 which covers a micro-biological chamber arrangement with particular configurations of access to the inside of the container. Other arrangements include, for example, U.S. Patent 3,702,806 which covers a triangular shaped culture media container, U.S. Patent 4,334,028 which covers a flask with an accessible opening in the top, U.S. Patent 3,449,210 which covers a specific flat bottom flask for culturing, and U.S. Patent 3,870,602 which covers a relatively square flat bottomed flask with an angled neck for gaining better access to the internal bottom surface of the flask. A further patent which covers and/or shows a configuration of culture flask is U.S. Design Patent 285725 which, again, shows an angled configuration of flask.

Each of these structures provides containers for developing pathogens of various kinds and they all have certain deficiencies in that each one of them has walls which are made separately and joined together and, therefore, are subject to failure and/or leakage. With the various biohazardous systems being examined these days it is most important to avoid any kind of leakage problem. For example, U.S. Patent 3,870,602 teaches that various parts thereof are sonically welded together. However, if there is an accident and such a flask is dropped from the shelves provided in a culture environment room, the flask may break and allow the escape of dangerous pathogens. The laboratory personnel, therefore, may be exposed to the dangerous pathogens and may, indeed, be affected by such pathogens.

With this invention, by contrast, an enlarged laboratory flask is provided which is blow molded. That is, the entire extent of the walls of the flask of the invention are developed simultaneously as a single integral piece, so that there are no seams and/or welds which might break as a result of accidental handling of such flasks. Moreover, the flask of the invention has been formed much larger than those in the past to provide a substantial flat bottom surface for culturing cells. The material utilized for the blow molding operation is such that the resulting flask is transparent throughout the extent thereof for ready examination through the walls of the flask as to the development of cultured cells therein.

A further feature of the invention is the incorporation into the side walls of the flask of the invention of a handle hold for easy gripping of the large flask of the invention. Thus, the flasks are much more easily manipulated, picked up and put down without being dropped, as discussed above.

A further feature of the invention is the fact that the top and bottom walls of each flask of the invention include cooperating surfaces for interlocking stacking of the individual flasks one upon each other. That is, the top surface includes abutments which cooperate with the bottom surface thereof to cause stacked individual flasks of the invention to be locked in the stacked position, so that individual flasks in a stack do not slide off of each other. This, of course, further prevents accidents and the potential of contamination from pathogens being developed in the flask.

Other objects and advantages of this invention will be apparent from the following description, the accompanying drawings and the appended claims.

### DESCRIPTION OF THE DRAWINGS

Fig. 1 is a top plan view of one embodiment illustrating the flask of the invention;
Fig. 2 is a side elevational view of the flask of Fig. 1;
Fig. 3 is one end view of the flask of Fig. 1;
Fig. 4 is a sectional view taken along lines 4-4 of Fig. 1;
Fig. 5 is a top plan view of a further embodiment illustrating the flask of the invention;
Fig. 6 is a bottom plan view of the flask of Fig. 5;
Fig. 7 is a side elevational view of the flask of Fig. 5;
Fig. 8 is an end view of one end of the flask of Fig. 5; and
Fig. 9 is a sectional view taken along lines 9-9 of Fig. 6.

### DETAILED DESCRIPTION OF THE INVENTION

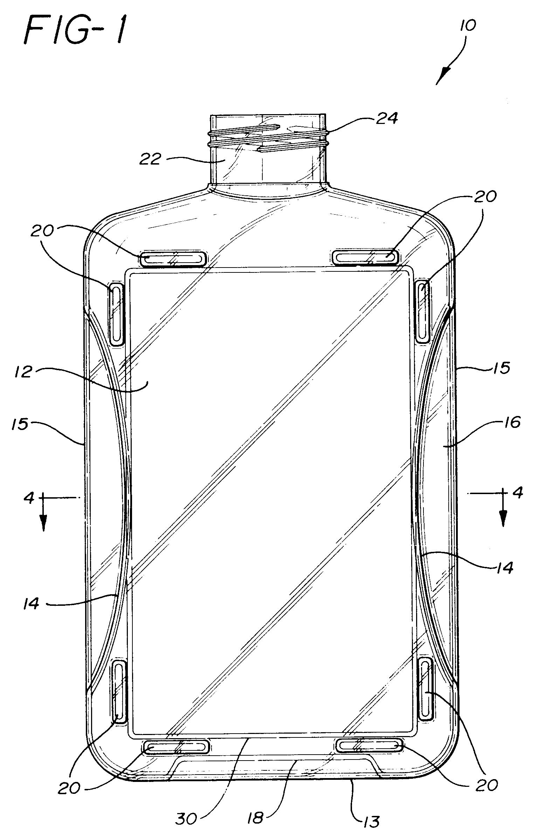

Referring to the drawings in which like reference characters refer to like parts throughout the several views thereof, Fig. 1 illustrates the invention as employed as a laboratory flask for culturing cells in a laboratory environment. As discussed above, cells of the kind herein will be developed in flasks, with a number of flasks positioned in a room with a specific environment. For example, ambient gases may be introduced into the flask if the caps are left loose. Tightened caps provide a closed environment.

In Fig. 1, the top plan view of one embodiment of laboratory flask is illustrated and designated generally 10, having a top surface 12 and opposed side walls 15. The flask includes wall 13, which is a closed end and, on the opposite end thereof, neck 22 providing access to the internal chamber of the flask 10. Neck 22 generally will include angled threads 24 for receiving a cap on neck 22. The cap may be the usual screw cap for sealing the internal chamber of flask 10. However, the cap may be configured like that taught in U.S. Patent 4,289,248 which allows the cap to have an intermediate positioning so as to allow leakage around the cap threads of gas in a room for allowing the gas to enter the chamber of the flask, during the development of cells therein.

As can be seen in Fig. 1, each side wall 15 of flask 10 includes indentations 14 which serve as opposed gripping surfaces for handling flask 10 during stacking and/or examination thereof. The indentations provide a flat surface 16 as shown in Fig. 1. Arranged at each corner of the top surface 12 of flask 10 are a pair of abutments 20 which serve to received the four corners of the bottom surface of a duplicate flask configured the same as flask 10. Thus, the individual abutments 20 at each corner serve to provide a stable stacking arrangement for individual flasks 10 stacked together on a shelf. As seen in Fig. 1, end surface 13 of flask 10 includes an indentation 18 which is formed during the blow molding procedure of flask 10, for holding it in place during the actual formation thereof.

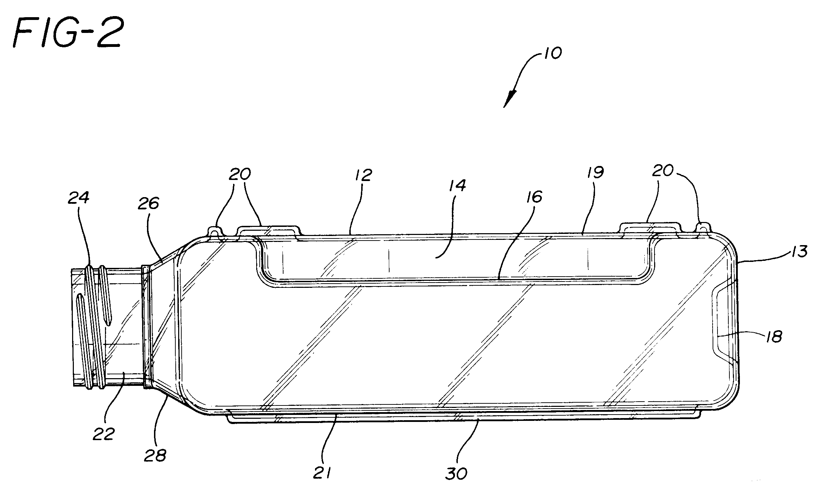

Fig. 2 shows a side elevational view of the flask 10 of Fig. 1. As can be seen on the left hand end of flask 10, the top and bottom walls 19, 21 of flask 10 converge toward the neck 22 as shown at 26, 28, respectively. The flat bottom wall 21 of flask 10 includes the bottom square extension 30 which cooperates with the corner abutments 20 on the top surface of flask 10.

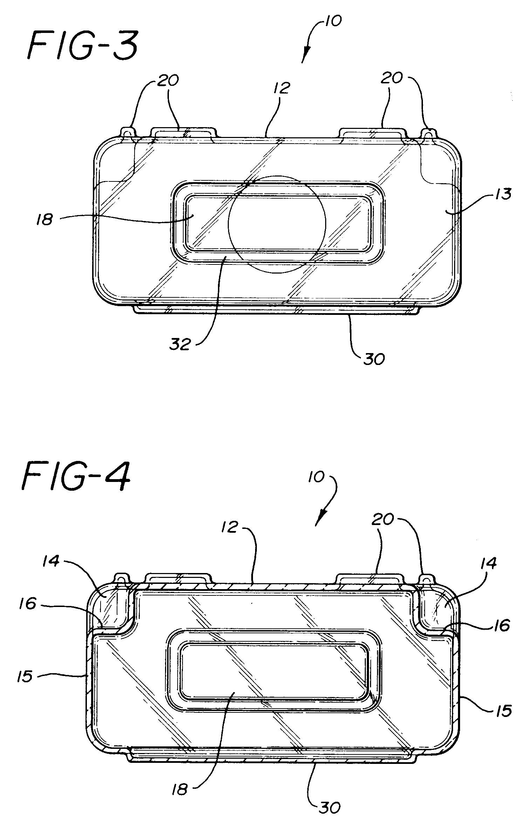

Fig. 3 shows the end view of flask 10 opposite neck 24, and shows the configuration of the indentation 18 formed during the molding process. Moreover, the square arrangement of the bottom flat surface 30 is shown in its end view in Fig. 3.

In Fig. 4, a sectional view of flask 10 of Fig. 1 is shown and indicates the flat square configuration of the flat surface 30 of flask 10 as well as the flat top surface 12 thereof, and the sectional view of the gripping surfaces 14 of flask 10.

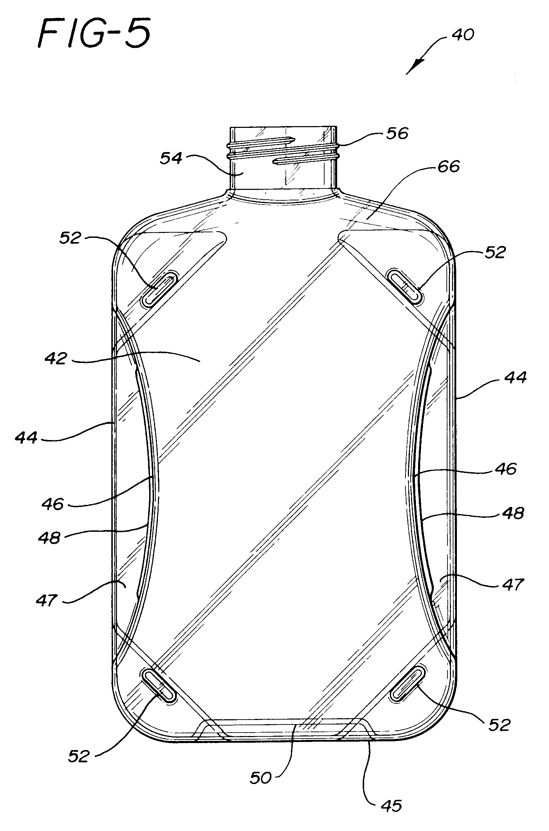

Referring now to Fig. 5, a further embodiment of the invention is shown. This flask, generally designated 40, includes a top surface 42 which has indented walls 46 for providing the gripping and/or handling surfaces. Each gripping surface 46 extends from the opposed side walls 44 of flask 40 in a manner similar to that of the embodiment illustrated in Figs. 1-4. The gripping indentations 46 provide horizontal surfaces 47 similar to the surfaces 16 in the flask described in Figs. 1-4.

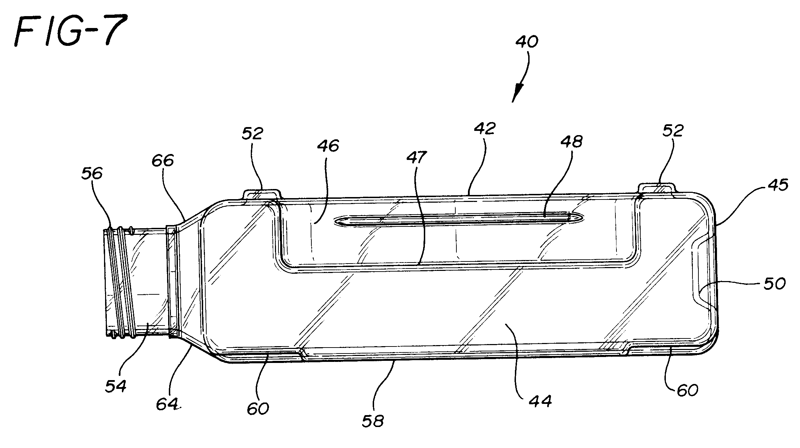

In this arrangement, the walls 46 forming the gripping surfaces for handling flask include a gripping ledge 48 shown in Fig. 5 and shown in more detail in Fig. 7 to be described below. Flask 40 includes a neck 54 with threads 56 in the same manner as the earlier described embodiment. Moreover, flask 40 includes an indentation 50 formed during the blow molding operation. The top surface of flask 40 includes abutments 52 angled at each corner of flask 40 for receiving angled corners to the flat surface formed on the bottom surface of flask 40 to be described in more detail below.

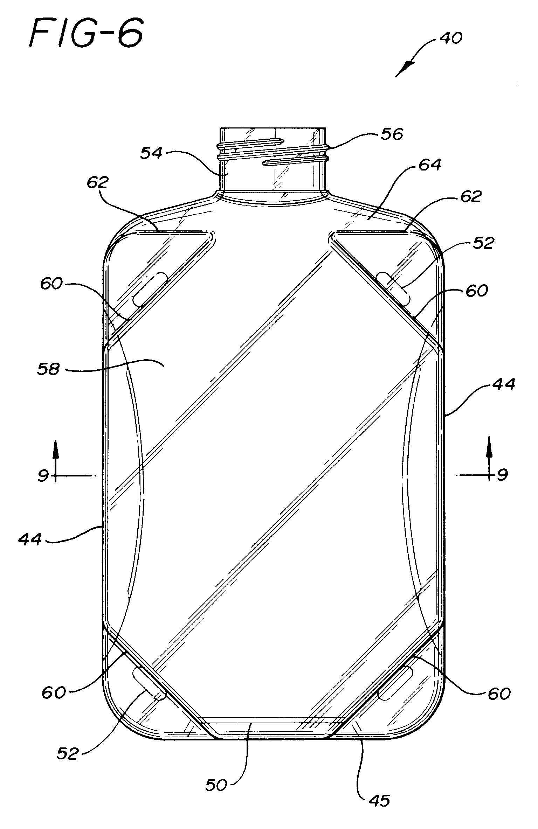

Referring now to Fig. 6, the bottom surface of flask 40 is shown in the form of a flat centrally positioned surface 58 having angled corners 60. These angled corners 60 fit up against the abutments 52 in the top surface of the flask 42 to provide the stacking arrangement for this particular embodiment of flask. Flask 40, includes an end wall 45 which has the insertion 50 therein formed during the molding process. Edges 62 are formed as a borderline between the flat surface 58 and the angled converging surface 64 converging from flat surface 58 to neck 54.

Referring now to Fig. 7, a side elevational view of the flask 40 is shown. The converging surface 64 is clearly shown as well as opposed converging surface 66 which converge from the flat surfaces 58, 42 toward neck 54. Flat bottom surface 58 is clearly shown in Fig. 7, with the side view of the angled indentations 60, as described above. The elongated gripping ledge 48 is clearly shown in this side elevational view extending along one side gripping surface 46.

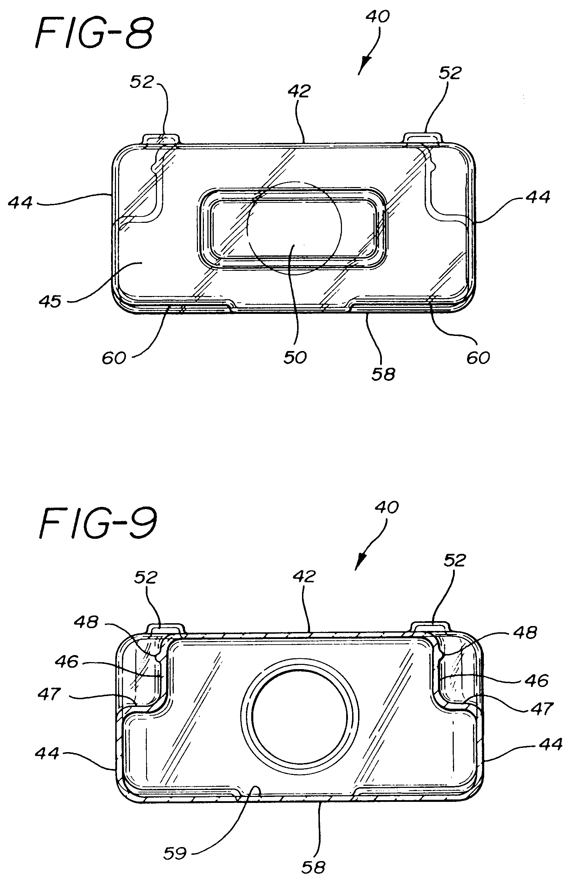

Referring to Fig. 8, an end view of flask 40 is shown with the rectangular shaped indentation 50 formed during the molding process. This end wall 45 shows the flat bottom surface 58 in an end view together with the angles 60 shown on either side thereof and cooperating abutments 52 on the top surface 42.

Referring now to Fig. 9, a sectional view of the flask 40 is shown indicating the opposed side walls 44 and the opposed top and bottom walls 42, 58, respectively, as well as the very large flat cell culturing surface 59. As can be seen in Fig. 9, side walls 44 include the indentations 46 for providing a handling a gripping surface on either side of flask 40 for the easy manipulation thereof. The surfaces 46, of course, include the gripping ledge 48.

Preferably, the flasks of the invention will be blow molded from polystyrene. Other representative materials which may be used are polyethylene terephthalate, polycarbonate or polypropylene.

As will be appreciated from the above discussion, the flask of the invention may be produced in substantial quantities by a blow molding procedure wherein the individual flasks obtained do not have any seams for subsequent failure and/or exposure to the user thereof to biohazardous agents which may be developed in the flask. Moreover, the individual flasks have substantial flat surfaces for developing large quantities of cells thereon with those flat surfaces, because of the large dimensions of the flask herein being readily available to the large opening provided at the neck of each flask.

Finally, the flasks are configured to have cooperating mating surfaces on the top and bottom surfaces thereof for enhancing the stackability for the flask for developing large quantities of cells in individual flasks of large numbers contained in a developed environment for that purpose. The materials, discussed above, for the blow molding operation are is selected to provide clear transparent flasks which may be examined readily to determine the condition and development of cells therein.

While the forms of apparatus herein described constitute preferred embodiments of the invention, it is to be understood that the invention is not limited to these precise forms of apparatus, and that changes may be made therein without departing from the scope of the invention which is defined in the appended claims.

## Claims

1. A culture flask comprising
(a) opposed top and bottom walls, said top and bottom walls being substantially flat and parallel to each other;
(b) a side wall forming each side of said flask, each said side wall connected to said top and bottom walls along the top and bottom edges of said side walls respectively;
(c) a rectangular closed end wall joining one end of said top and bottom walls and said side walls and forming one end of said flask;
(d) an externally threaded tubular neck on the end of said flask opposite said closed end wall;
(e) said side walls and said top and bottom walls converging gradually toward said tubular neck; and
(f) said top wall, said bottom wall, said side walls, said end wall and said neck being formed simultaneously by blow molding to form a seamless culture flask.

2. The culture flask of Claim 1, further comprising
(a) an elongated indentation in each said side wall;
(b) each said indentation giving each said side wall a stepped configuration in cross section; and
(c) said opposed indentations providing gripping surfaces for holding said culture flask.

3. The culture flask of Claim 2, further comprising
(a) a gripping ledge extending along each said elongated indentation in each said side wall;
(b) said gripping ledge enhancing the gripping of said culture flask for the gripping thereof.

4. The culture flask of Claim 1, further comprising
(a) a flat rectangular extension extending integrally from and forming the major portion of said bottom wall;
(b) a pair of abutments extending integrally from each corner of said top wall; and
(c) each pair of abutments being positioned at right angles to each other;
(d) whereby a flat rectangular extension on the bottom wall of one culture flask fits in and may be contained by the four pairs of abutments positioned on the top surface of another culture flask for stacking a plurality of said flasks.

5. The culture flask of Claim 1, further comprising
(a) a flat generally rectangular extension extending integrally from and forming the major portion of said bottom wall;
(b) said generally rectangular extension having an angular wall at each of the four corners thereof whereby said generally rectangular extension is modified into an octagonal shape;
(c) an abutment extending integrally from each corner of said top wall;
(d) each said integral abutment being angled at 180° from the said side walls of said culture flask;
(e) whereby each of said angular abutments on the said top wall of one of said culture flasks abuts against an angular corner wall of said generally rectangular extension of said bottom wall of another flask for stacking a plurality of said flasks.

6. A culture flask comprising
(a) opposed top and bottom walls, said top and bottom walls being substantially flat and parallel to each other;
(b) a side wall forming each side of said flask, each said side wall connected to said top and bottom walls along the top and bottom edges of said side walls, respectively;
(c) a rectangular closed end wall joining one end of said top and bottom walls and said side walls and forming one end of said flask;
(d) an externally threaded tubular neck, on the end of said flask opposite said closed end wall;
(e) said side walls and said top and bottom walls converging gradually toward said tubular neck;
(f) an elongated indentation in each said side wall;
(g) each said indentation giving each said side wall a stepped configuration in cross-section;
(h) said opposed indentations providing gripping surfaces for holding said culture flask;
(i) a flat rectangular extension extending integrally from and forming the major portion of said bottom wall;
(j) a pair of abutments extending integrally from each corner of said top wall; and
(k) each pair of abutments being positioned at right angles to each other;
(l) whereby a flat rectangular extension on the bottom wall of one culture flask may fit in and is contained by the four pairs of abutments positioned on the top surface of another culture flask for stacking a plurality of said flasks.

7. The culture flask of Claim 6, further comprising
(a) said top wall, said bottom wall, said side walls, said end wall, and said neck are formed simultaneously by blow molding to form a seamless culture flask.

8. The culture flask of Claim 6, further comprising
(a) a gripping ledge extending along each said elongated indentation in each said side wall;
(b) said gripping ledge enhancing the gripping of said culture flask for the gripping thereof.

9. A culture flask comprising
(a) opposed top and bottom walls, said top and bottom walls being substantially flat and parallel to each other;
(b) a side wall forming each side of said flask, each said side wall connected to said top and bottom walls along the top and bottom edges of said side walls, respectively;
(c) a rectangular closed end wall joining one end of said top and bottom walls and said side walls and forming one end of said flask;
(d) an externally threaded tubular neck, on the end of said flask opposite said closed end wall;
(e) said side walls and said top and bottom walls converging gradually toward said tubular neck;
(f) an elongated indentation in each said side wall;
(g) each said indentation giving each said side wall a stepped configuration in cross-section;
(h) said opposed indentations providing gripping surfaces for holding said culture flask;
(i) a flat generally rectangular extension extending integrally from and forming the major portion of said bottom wall;
(j) said generally rectangular extension having an angular wall at each of the four corners thereof whereby said generally rectangular extension is modified into an octagonal shape;
(k) an abutment extending integrally from each corner of said top wall; and
(l) each said integral abutment being angled at 180° from said side walls of said culture flask;
(m) whereby each of said angular abutments on the said top wall of one of said culture flasks abuts against an angular corner wall of said generally rectangular extension of said bottom wall of another flask.

10. The culture flask of Claim 9, further comprising
(a) said top wall, said bottom wall, said side walls, said end wall, and said neck are formed simultaneously by blow molding to form a seamless culture flask.
